(19) Europäisches Patentamt

European Patent Office

Office européen des brevets

(11) EP 1 866 667 B1

(12) FASCICULE DE BREVET EUROPEEN

(45) Date de publication et mention de la délivrance du brevet:
26.02.2014 Bulletin 2014/09

(21) Numéro de dépôt: 06726152.9

(22) Date de dépôt: 30.03.2006

(51) Int Cl.:
*G01S 15/89* (2006.01)          *A61B 8/08* (2006.01)
*G01S 7/52* (2006.01)

(86) Numéro de dépôt international:
PCT/FR2006/000702

(87) Numéro de publication internationale:
WO 2006/106213 (12.10.2006 Gazette 2006/41)

(54) **PROCEDE ET DISPOSITIF D IMAGERIE UTILISANT DES ONDES DE CISSAILLEMENT**

SCHERWELLEN VERWENDENDES ABBILDUNGSVERFAHREN UND EINRICHTUNG

IMAGING METHOD AND DEVICE USING SHEARING WAVES

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **05.04.2005 FR 0503376**

(43) Date de publication de la demande:
**19.12.2007 Bulletin 2007/51**

(73) Titulaire: **SUPER SONIC IMAGINE**
**13857 Aix-en-Provence (FR)**

(72) Inventeurs:
• **FINK, Mathias**
**F-92190 Meudon (FR)**
• **SINKUS, Ralph**
**F-75018 Paris (FR)**
• **TANTER, Mickael**
**F-75006 Paris (FR)**

• **BERCOFF, Jeremy**
**F-75013 Paris (FR)**

(74) Mandataire: **Cabinet Plasseraud**
**52, rue de la Victoire**
**75440 Paris Cedex 09 (FR)**

(56) Documents cités:
WO-A-2004/021038          US-A- 5 606 971
US-A- 5 810 731          US-A1- 2004 204 860
US-A1- 2005 038 339          US-B1- 6 791 901

• TANTER M ET AL: "Ultrafast compound imaging for 2-D motion vector estimation: application to transient elastography" IEEE TRANSACTIONS ON ULTRASONICS, FERROELECTRICS AND FREQUENCY CONTROL IEEE USA, vol. 49, no. 10, octobre 2002 (2002-10), pages 1363-1374, XP002354376 cité dans la demande

**Description**

La présente invention est relative aux procédés et dispositifs d'imagerie utilisant des ondes de cisaillement.

Plus particulièrement, l'invention concerne un procédé d'imagerie dans lequel on génère, dans un milieu viscoélastique, une onde mécanique ayant une composante de cisaillement et une composante de compression, et on détermine au moins un paramètre de mouvement du milieu viscoélastique en différents points lors de la propagation de cette onde mécanique, ce procédé comportant au moins une étape d'observation au cours de laquelle on observe la propagation de l'onde mécanique en une multitude de points du milieu viscoélastique, par une technique choisie parmi l'échographie ultrasonore et la résonance magnétique.

On permet ainsi une analyse qualitative et/ou quantitative, notamment pour repérer des zones de dureté différente du reste du milieu viscoélastique ou des zones ayant un temps de relaxation différent du reste du milieu viscoélastique.

Le document WO-A-04/21 038 décrit un exemple d'un tel procédé, dans lequel on détermine ledit paramètre de mouvement (notamment le déplacement) dans un plan à la suite de la génération à distance d'une poussée localisée impulsionnelle qui crée l'onde mécanique en question, en l'occurrence une onde de cisaillement.

Bien que ce procédé donne déjà toute satisfaction, la présente invention a pour but de le perfectionner encore, de façon à en améliorer la précision et à permettre le cas échéant de l'utiliser pour de l'imagerie en trois dimensions.

A cet effet, un procédé du genre en question est caractérisé en ce que l'onde mécanique est entretenue et en ce qu'il comporte une étape de correction au cours de laquelle on traite le paramètre de mouvement pour éliminer les effets dus à la composante de compression de ladite onde mécanique et pour ne prendre en compte que les effets dus à la composante de cisaillement de ladite onde mécanique.

Ainsi, lorsqu'on utilise une onde mécanique non plus impulsionnelle mais entretenue, par exemple pour effectuer une mesure relativement longue telle que pour de l'imagerie en trois dimensions, la composante de compression de l'onde mécanique est toujours présente au moment de la mesure du paramètre de mouvement et perturbe donc fortement cette mesure : le traitement selon l'invention permet d'éliminer cette perturbation, et de déterminer le cas échéant, une cartographie en deux ou en trois dimensions des paramètres viscoélastiques du milieu.

Dans des modes de réalisation préférés du procédé selon l'invention, on peut éventuellement avoir recours en outre à l'une et/ou à l'autre des dispositions suivante :

- au cours de ladite étape de correction, on calcule un rotationnel dudit paramètre de mouvement ;
- l'étape d'observation (b) comprend les sous-étapes suivantes :

  (b1) faire émettre dans le milieu viscoélastique, par un réseau de transducteurs, une succession de tirs d'ondes ultrasonores de compression (focalisées ou non focalisées) à une cadence d'au moins 10 tirs par seconde,
  (b2) faire détecter et enregistrer en temps réel des signaux acoustiques reçus du milieu viscoélastique, comprenant les échos générés par les ondes ultrasonores de compression en interagissant avec le milieu viscoélastique ;

- le procédé comporte en outre au moins une étape de traitement (c) au cours de laquelle :

  (c1) on traite les signaux acoustiques successifs reçus du milieu viscoélastique au cours de la sous-étape (b2) pour déterminer des images de propagation successives de l'onde mécanique,
  (c2) et on détermine ledit paramètre de mouvement du milieu viscoélastique en différents points à partir desdites images de propagation successives ;

- on réalise plusieurs images successives de propagation de l'onde mécanique dans le milieu viscoélastique selon plusieurs points de vue, et on combine ces images successives pour déterminer une valeur vectorielle dudit paramètre de mouvement ;
- on détermine une valeur vectorielle à trois dimensions dudit paramètre de mouvement ;
- pour réaliser les images successives de propagation de l'onde mécanique selon plusieurs points de vue, on utilise un réseau de transducteurs ultrasonores (réseau linéaire ou tout autre réseau, tel que "Phased Array", ou réseau 1.5D, réseau 1.75D ou encore matrice 2D de transducteurs piézoélectriques) qui émet et détecte les ondes ultrasonores de compression selon une direction d'émission et détection dans un plan d'analyse, et l'on déplace ledit réseau à l'extérieur du milieu viscoélastique en faisant tourner ledit plan d'analyse (ces dispositions pourraient éventuellement être utilisées indépendamment des dispositions qui précèdent, et l'invention a donc également pour objet ces dispositions en tant que telles) ;
- on déplace le réseau en translation sans faire tourner le plan d'analyse, puis on fait tourner ledit réseau en faisant tourner ledit plan d'analyse ;

- on fait varier la direction d'émission et réception des transducteurs du réseau sans faire tourner le plan d'analyse, puis on fait tourner ledit réseau en faisant tourner ledit plan d'analyse ;
- ledit paramètre de mouvement est choisi parmi un déplacement, une vitesse de déplacement et une déformation du milieu viscoélastique ;
- le procédé comprend une étape (d) de cartographie au cours de laquelle, à partir d'une évolution du paramètre de mouvement au cours du temps, on calcule au moins un paramètre de propagation de la composante de cisaillement de l'onde mécanique en au moins certains points du milieu viscoélastique pour déterminer ainsi une cartographie dudit paramètre de propagation dans le milieu viscoélastique ;
- le paramètre de propagation de l'onde de cisaillement qui est calculé au cours de l'étape de cartographie, est choisi parmi la vitesse des ondes de cisaillement, le module de cisaillement, le module d'Young, l'atténuation des ondes de cisaillement, l'élasticité de cisaillement, la viscosité de cisaillement et le temps de relaxation mécanique, ou plusieurs de ces paramètres dans le cas d'un milieu anisotrope ;
- le milieu viscoélastique qui est un corps vivant.

Par ailleurs, l'invention a également pour objet un dispositif d'imagerie comprenant des moyens pour générer, dans un milieu viscoélastique, une onde mécanique ayant une composante de cisaillement et une composante de compression, et des moyens pour déterminer au moins un paramètre de mouvement du milieu viscoélastique en différents points lors de la propagation de cette onde mécanique, le dispositif comportant des moyens d'observation pour observer la propagation de l'onde mécanique en une multitude de points du milieu viscoélastique, par une technique choisie parmi l'échographie ultrasonore et la résonance magnétique, et
caractérisé en ce que l'onde mécanique est entretenue et en ce qu'il comporte des moyens de correction pour traiter le paramètre de mouvement pour éliminer les effets dus à la composante de compression de ladite onde mécanique et pour ne prendre en compte que les effets dus à la composante de cisaillement de ladite onde mécanique.
Dans des modes de réalisation préférés du dispositif selon l'invention, on peut éventuellement avoir recours en outre à l'une et/ou à l'autre des dispositions suivantes :

- les moyens d'observation comportent un réseau de transducteurs ultrasonores pour observer la propagation de l'onde mécanique en une multitude de points du milieu viscoélastique, par échographie ;
- les moyens de correction sont adaptés pour calculer un rotationnel dudit paramètre de mouvement ;
- le dispositif comporte des moyens pour :

    (b1) faire émettre dans le milieu viscoélastique, par ledit réseau de transducteurs, une succession de tirs d'ondes ultrasonores de compression (focalisées ou non focalisées) à une cadence d'au moins 10 tirs par seconde,
    (b2) faire détecter et enregistrer en temps réel des signaux acoustiques reçus du milieu viscoélastique, comprenant les échos générés par les ondes ultrasonores de compression non focalisées en interagissant avec les particules réfléchissantes dudit milieu viscoélastique ;

- le dispositif comporte des moyens de traitement adaptés pour :

    (c1) traiter les signaux acoustiques successifs reçus du milieu viscoélastique pour déterminer des images de propagation successives de l'onde mécanique,
    (c2) et déterminer ledit paramètre de mouvement du milieu viscoélastique en différents points du champ d'observation à partir desdites images de propagation successives ;

- les moyens de traitement sont adaptés pour réaliser plusieurs images successives de propagation de l'onde mécanique dans le milieu viscoélastique selon plusieurs points de vue, pour combiner ces images successives en déterminant une valeur vectorielle dudit paramètre de mouvement ;
- les moyens de traitement sont adaptés pour déterminer une valeur vectorielle à trois dimensions dudit paramètre de mouvement ;
- le réseau de transducteurs émet et détecte les ondes ultrasonores de compression selon une direction d'émission et détection dans un plan d'analyse et, pour réaliser les images successives de propagation de l'onde mécanique selon plusieurs points de vue, le dispositif comporte des moyens de déplacement dudit réseau (réseau linéaire ou tout autre réseau, tel que "Phased Array", ou réseau 1.5D, réseau 1 .75D ou encore matrice 2D de transducteurs piézoélectriques) à l'extérieur du milieu viscoélastique, adaptés pour faire tourner ledit plan d'analyse (ces dispositions pourraient éventuellement être utilisées indépendamment des dispositions qui précèdent, et l'invention a donc également pour objet ces dispositions en tant que telles) ;
- le dispositif comporte des moyens de commande adaptés pour commander les moyens de déplacement et lesdits moyens de commande sont adaptés pour déplacer le réseau en translation sans faire tourner le plan d'analyse, puis pour faire tourner ledit réseau en faisant tourner ledit plan d'analyse ;

- le dispositif comporte des moyens de commande adaptés pour commander les moyens de déplacement et lesdits moyens de commande sont adaptés pour faire varier la direction d'émission et réception des transducteurs du réseau sans faire translater et tourner le plan d'analyse, puis pour faire translater et tourner ledit réseau en faisant tourner ledit plan d'analyse ;
- le dispositif comporte des moyens de cartographie adaptés pour calculer au moins un paramètre de propagation de la composante de cisaillement de l'onde mécanique en au moins certains points du milieu viscoélastique pour déterminer ainsi une cartographie dudit paramètre de propagation dans le milieu viscoélastique.

D'autres caractéristiques et avantages de l'invention apparaîtront au cours de la description suivante d'une de ses formes de réalisation, donnée à titre d'exemple non limitatif, en regard des dessins joints.

Sur les dessins :

- la figure 1 est une vue schématique d'un dispositif d'imagerie par ondes de cisaillement selon une forme de réalisation de l'invention,
- la figure 2 est une vue de détail en perspective d'une partie du dispositif de la figure 1,
- et la figure 3 est un schéma bloc du dispositif de la figure 1.

Sur les différentes figures, les mêmes références désignent des éléments identiques ou similaires.

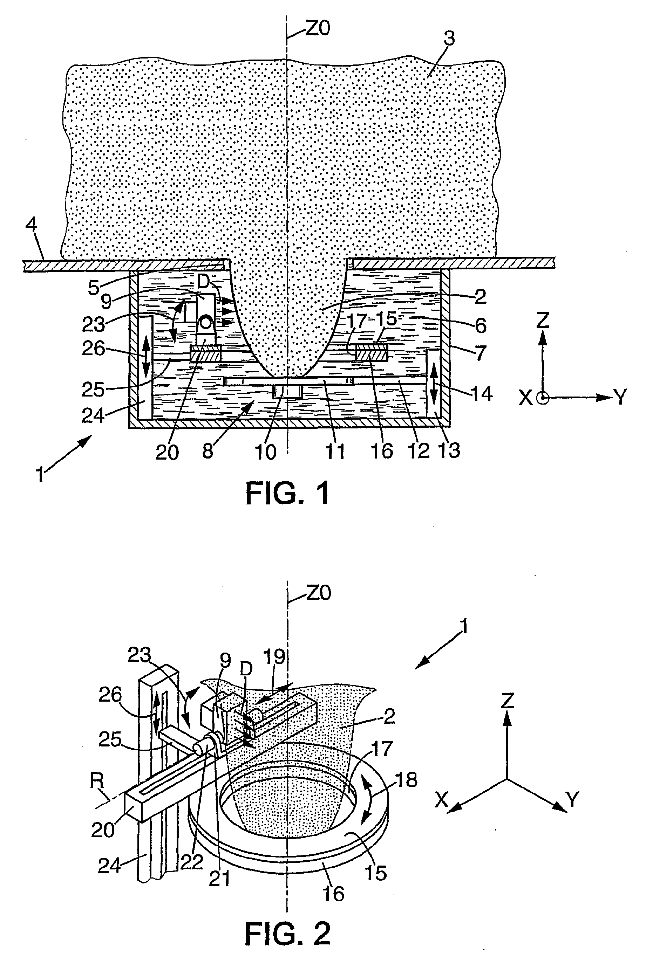

Le dispositif d'imagerie 1 représenté sur la figure 1 est destiné à étudier la propagation des ondes élastiques de cisaillement dans un milieu viscoélastique 2 qui est diffusant vis à vis des ondes ultrasonores de compression, et qui peut être par exemple :

- un corps inerte, notamment dans le cas du contrôle de qualité pour des applications industrielles,
- ou un corps vivant, par exemple une partie du corps d'un patient, dans le cas des applications médicales.

Dans l'exemple représenté sur les dessins, le dispositif d'imagerie 1 est plus spécialement adapté pour l'imagerie médicale du sein d'une patiente 3, le sein constituant alors le milieu 2 à imager.

A cet effet, le dispositif d'imagerie 1 peut par exemple comporter une table d'examen 4 sur laquelle la patiente peut se coucher sur le ventre. Cette table est pourvue d'un évidement 5 laissant le passage pour au moins un sein 2 de la patiente qui pend dans un bain 6 de produit couplant ultrasonore, par exemple gel ou liquide, de préférence de l'eau. Ce bain de liquide 6 est délimité par un récipient 7 qui contient également un réseau 9 de transducteurs ultrasonores et un générateur de vibrations 8 en contact avec le sein. Le récipient 7 peut être transparent pour permettre notamment un contrôle du bon positionnement du générateur de vibrations 8 contre le sein 2 et du réseau de transducteurs 9 sur le côté du sein.

On notera que la table d'examen 4 peut être le cas échéant adaptée pour laisser la passage aux deux seins de la patiente qui pendent alors dans le même bain de liquide 6 ou dans deux bains différents. Le dispositif d'imagerie peut alors éventuellement être adapté pour imager simultanément les deux seins de la patiente.

Le générateur de vibrations 8 peut comprendre un transducteur acoustique 10 qui génère des vibrations mécaniques basse fréquence dans une plaque 11 de hauteur réglable, positionnée par exemple au contact avec l'extrémité du sein 2 de la patiente pour y transmettre des ondes mécaniques (élastiques), notamment des ondes entretenues comprenant une composante compression et une composante de cisaillement. Les ondes mécaniques en question peuvent être de fréquence comprise par exemple entre 0.1 Hz et 10 kHz.

On notera que les ondes mécaniques susmentionnées pourraient être générées autrement que par le générateur de vibrations susmentionné, par exemple :

- par un transducteur générant des ondes basses fréquence et positionné différemment contre le sein 2,
- ou par un réseau de transducteurs ultrasonores générant des ondes mécaniques basse fréquence à distance au moyen d'ondes ultrasonores focalisées modulées dans le temps, en utilisant la pression de radiation comme expliqué par exemple dans le document WO-A-04/21 038 susmentionné ou dans le document US-A-5 606 971.

Dans l'exemple représenté sur la figure 1, la hauteur de la plaque 11 est réglable pour venir au contacte du sein 2. Ce réglage peut éventuellement être motorisé, par exemple au moyen d'un vérin à vis 13 à moteur électrique pas à pas étanche ou autre. Le vérin 13 peut être relié à la plaque 11 par exemple par une tringle de support 12, pour déplacer la plaque 11 et le transducteur acoustique 10 dans le sens de la double flèche 14, selon un axe vertical Z.

Comme représenté sur les figures 1 et 2, le réseau 9 de transducteurs peut par exemple être un réseau linéaire en forme de barrette, dans lequel les transducteurs sont alignés dans le plan vertical et sont adaptés pour émettre et capter des ondes ultrasonores tous parallèlement à une même direction d'émission et détection D, dans un plan d'analyse vertical (D, Z).

Le réseau 9 de transducteurs peut être monté par exemple sur un plateau tournant 15 porté par un plateau fixe 16. Les deux plateaux 15, 16 s'étendent dans le plan horizontal (X, Y) et sont avantageusement évidés pour laisser libre un passage central 17 traversé par le sein 2 de la patiente. Le plateau tournant 15 est déplaçable angulairement par rapport au plateau fixe 16, autour d'un axe central vertical Z0, au moyen d'un système de réglage à moteur électrique pas à pas étanche (non visible sur les figures 1 et 2) pouvant être par exemple intégré entre les plateaux 15 et 16. En déplaçant le plateau tournant 15, on fait donc tourner le plan d'analyse (D, Z), ce qui permet d'imager le sein 2 selon plusieurs points de vue, notamment en vue de reconstituer une image à trois dimensions comme il sera expliqué ci-après.

Le réseau 9 de transducteurs peut en outre être déplaçable en translation dans la direction de la double flèche 19, perpendiculairement au plan d'analyse (D, Z), pour imager plusieurs plans d'analyse parallèles (par exemple environ 30 plans d'analyse) avant de faire tourner ledit plan d'analyse. A cet effet, le réseau de transducteurs peut par exemple être monté coulissant dans la direction 19 sur une réglette transversale 20 qui s'étend selon une direction orthoradiale à l'axe central Z0 et qui est fixée au plateau tournant 15. Le réseau 9 de transducteurs est déplaçable le long de la réglette 20 par exemple au moyen d'un système de réglage à moteur pas à pas étanche commandant un ensemble vis - écrou intégré dans la réglette 20.

Le réseau 9 de transducteur peut le cas échéant être relié à la réglette 20 par un tourillon 21 qui coulisse le long de ladite réglette et sur lequel le réseau 9 peut pivoter autour d'un axe de rotation R parallèle à la direction 19 susmentionnée. On peut ainsi, éventuellement, faire varier la direction D en site, c'est-à-dire faire varier l'inclinaison de la direction D par rapport au plan horizontal (X, Y) sans faire varier le plan d'analyse (D, Z). Comme il sera expliqué ci-après, ce mouvement éventuel permet également de varier les points de vue selon lesquels on image le sein 2, de manière à en reconstituer une image en trois dimensions.

Le pivotement du réseau 9 de transducteurs est par exemple commandé par un moteur électrique pas à pas étanche 22, qui permet de déplacer le réseau 9 dans la direction de la double flèche 23.

Eventuellement, le plateau fixe 16 peut par ailleurs être déplaçable verticalement pour imager le sein à plusieurs hauteurs, notamment si le réseau 9 de transducteurs n'est pas assez long pour couvrir la hauteur de champ d'observation souhaitée dans le sein 2.

Dans l'exemple représenté, la hauteur du plateau fixe 16 est réglable par exemple au moyen d'un vérin à vis 24 à moteur électrique pas à pas étanche ou autre, relié au plateau 16 par exemple par une tringle de support 25, pour déplacer le plateau 16 dans le sens de la double flèche 26, selon l'axe vertical Z.

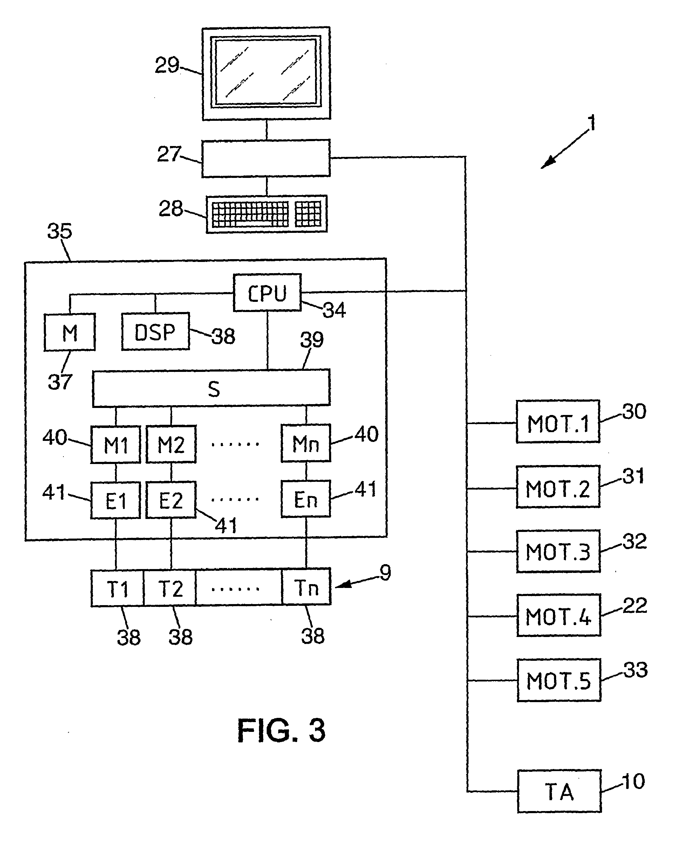

Comme représenté sur la figure 3, le dispositif d'imagerie peut en outre comporter un micro-ordinateur 27 (comprenant au moins une interface d'entrée 28 telle qu'un clavier ou autre, et une interface de sortie 29 telle qu'un écran ou autre) ou toute autre unité centrale électronique, qui commande :

- le transducteur acoustique 10 (TA),
- le moteur 30 (Mot. 1) du vérin 13,
- le moteur 31 (Mot. 2) commandant la rotation du plateau tournant 15,
- le moteur 32 (Mot. 3) commandant le coulissement du réseau 9 de transducteurs dans la direction 19,
- le moteur 22 (Mot. 4) commandant le pivotement du réseau 9 de transducteurs dans la direction 23,
- le moteur 33 (Mot. 5) du vérin 24,
- l'unité centrale électronique 34 (CPU) - microprocesseur ou autre - d'une baie électronique 35 de traitement du signal.

L'unité centrale 34 commande également, les n transducteurs 36 (T1, T2, ... Tn) du réseau 9 de transducteur (n peut par exemple être égal à 128 ou plus).

Eventuellement, la baie 35 peut comprendre également :

- une mémoire 37 (M) reliée à l'unité centrale 34,
- un circuit spécialisé 38 (DSP) de traitement du signal, également relié à l'unité centrale 34,
- un sommateur 39 (S) relié à l'unité centrale 34,
- n mémoires 40 (M1, M2, ... Mn) reliées au sommateur et affectées chacune à un transducteur 36 (chaque mémoire 40 peut par exemple avoir une capacité de 128 Mo ou plus),
- n échantillonneurs 41 (E1, E2, ... En) interposés entre chaque transducteur 36 et la mémoire 40 correspondante.

Le dispositif qui vient d'être décrit fonctionne comme suit.

Le micro-ordinateur 27 fait générer des ondes mécaniques élastiques entretenues dans le milieu viscoélastique 2 par le transducteur acoustique 10, au cours d'une étape d'excitation (a).

Par ailleurs, on observe la propagation de l'onde mécanique dans le milieu viscoélastique 2 par échographie, au cours d'une étape d'observation (b) concomitante à l'étape d'excitation (a). A cet effet, le micro-ordinateur 27 fait également envoyer des ondes ultrasonores de compression (à une fréquence comprise par exemple entre 0,5 et 100 MHz et de préférence entre 0,5 et 15 MHz, par exemple de l'ordre de 4 MHz) par les transducteurs 36 du réseau 9.

Ces ondes de compression, après passage dans le bain de gel couplant ou liquide 6, pénètrent dans le milieu 2 où elles se réfléchissent sur des particules diffusantes contenues dans ledit milieu 2, ce qui permet de suivre les mouvements du milieu 2. Les particules diffusantes en question peuvent être constituées par toute hétérogénéité du milieu 2, et notamment, lorsqu'il s'agit d'une application médicale, par des particules de collagène présentes dans les tissus humains.

Les ondes de compression ainsi émises peuvent être par exemple des ondes ultrasonores de compression "planes" (c'est à dire en l'occurrence une onde dont le front d'onde est rectiligne dans le plan d'analyse (D, Z)) ou tout autre type d'onde focalisée ou non focalisée éclairant l'ensemble du champ d'observation dans le milieu 2, par exemple une onde générée en faisant émettre des signaux acoustiques aléatoires par les différents transducteurs 36.

L'étape d'observation (b) susmentionnée peut comprendre les sous-étapes suivantes, pour chaque position du réseau 9 de transducteurs :

(b1) le micro-ordinateur 27 fait émettre dans le milieu viscoélastique 2, par le réseau 9, une succession de tirs d'ondes ultrasonores de compression,

(b2) le micro-ordinateur 27 fait détecter par le réseau 9 et enregistrer en temps réel des signaux acoustiques reçus du milieu viscoélastique 2, comprenant les échos générés par les ondes ultrasonores de compression en interagissant avec les particules diffusantes du milieu viscoélastique 2, ces échos correspondant (directement ou indirectement) à des images successives de déplacement du milieu viscoélastique.

Au cours de l'étape (b1), on peut émettre les ondes ultrasonores de compression, à une cadence comprise entre 10 et 10 000 tirs par seconde et de préférence comprise entre 10 et 5 000 tirs par seconde (cette cadence est limitée par le temps d'aller-retour de l'onde de compression dans le milieu 2, donc par l'épaisseur du milieu 2 dans la direction D : il faut en effet que tous les échos générés par l'onde de compression, aient été reçus par la sonde 6 avant d'envoyer une nouvelle onde de compression).

Chaque onde ultrasonore de compression se propage dans le milieu 2 avec une vitesse de propagation beaucoup plus élevée que les ondes de cisaillement (par exemple de l'ordre de 1500 m/s dans le corps humain), et interagit avec les particules diffusantes du milieu 2, ce qui génère des échos ou autres perturbations analogues du signal, connus en soi sous le nom de "bruits de speckle" dans le domaine de l'échographie.

Ces "bruits de speckle" sont captés par les transducteurs T1, ... ,Tn au cours de la sous-étape (b2), après chaque tir d'onde ultrasonore de compression. Le signal sij(t) ainsi capté par chaque transducteur Ti après le tir n° j est tout d'abord échantillonné à haute fréquence (par exemple de 30 à 100 MHz) et numérisé en temps réel (par exemple sur 12 bits) par l'échantillonneur correspondant Ei.

Le signal sij(t) ainsi échantillonné et numérisé est ensuite mémorisé, également en temps réel, dans la mémoire correspondante Mi.

Toujours pour chaque position du réseau 9 de transducteurs, le micro-ordinateur 27 procède ensuite, généralement en temps différé, à une étape de traitement (c) au cours de laquelle :

(c1) le micro-ordinateur 27 traite les signaux acoustiques successifs reçus du milieu viscoélastique 2 au cours de la sous-étape (b2) pour déterminer des images de propagation successives,

(c2) et le micro-ordinateur 27 détermine au moins un paramètre de mouvement (par exemple le déplacement ou la vitesse de déplacement) du milieu viscoélastique 2 en différents points du champ d'observation.

Au cours de la sous-étape (b2), après la mémorisation de tous les signaux sij (t) correspondant à une mesure dans une même position du réseau 9 de transducteurs, l'unité centrale 34 fait retraiter ces signaux par le circuit sommateur S (ou bien elle effectue elle-même ce traitement, ou encore ledit traitement peut être effectué dans le micro-ordinateur 27), par un processus classique de formation de voies correspondant à la sous-étape (c1).

On génère ainsi des signaux Sj (r, z) qui correspondent chacun à l'image du champ d'observation dans le plan (D, Z) après le tir n° j.

Par exemple, on peut déterminer un signal Sj (t) par la formule suivante :

$$Sj(t) = \sum_{i=1}^{n} \alpha_i(r, z).sij[t(r, z) + d_i(d, z)/Vr$$

où :

- sij est le signal brut perçu par le transducteur n° i après le tir d'onde ultrasonore de compression n° j,
- t(r,z) est le temps mis par l'onde ultrasonore de compression pour atteindre le point du champ d'observation de coordonnées (r,z), avec t = 0 au début du tir n° j, r étant une abscisse mesurée dans la direction D depuis une origine et z étant une ordonnée verticale du point considéré,
- di(r,z) est la distance entre le point du champ d'observation de coordonnées (r,z) et le transducteur n° i, ou une approximation de cette distance,
- V est la vitesse moyenne de propagation des ondes acoustiques ultrasonores de compression dans le milieu viscoélastique observé.
- et $\alpha i(r,z)$ est un coefficient de pondération tenant compte de lois d'apodisation (en pratique, on pourra dans de nombreux cas considérer que $\alpha i(r,z)=1$).

Après l'étape éventuelle de formation de voies, l'unité centrale 34 mémorise dans une mémoire centrale M appartenant à la baie 7 les signaux d'images Sj(r,z), où j est le numéro du tir d'onde de compression. Ces signaux peuvent également être mémorisés dans le micro-ordinateur 4 lorsqu'il effectue lui-même le traitement d'image.

Ces images sont traitées en temps différé ou immédiatement à la sous-étape (c2), par corrélation et avantageusement par intercorrélation soit deux à deux, soit de préférence avec une image de référence qui peut être :

- soit une image de déplacement précédemment déterminée comme expliqué ci-dessus et utilisée comme image de référence pour les images de déplacement ultérieures (ou pour un nombre limité d'images de déplacement ultérieures, par exemple 30 images de déplacement),
- soit une image déterminée au cours d'une étape préliminaire d'observation initiale (a0), de la même façon que les images successives de déplacement susmentionnées, en faisant émettre une ou plusieurs ondes ultrasonores par le réseau 9 de transducteurs avant l'étape d'excitation (a) qui génère l'onde de cisaillement (lorsque plusieurs ondes ultrasonores de compression sont ainsi émises avant la phase d'excitation, on enregistre des échos générés par chaque onde ultrasonore de compression en interagissant avec les particules réfléchissantes du milieu viscoélastique, ces échos correspondant à plusieurs images préliminaires successives du milieu viscoélastique, et on détermine ladite image initiale du milieu viscoélastique en combinant lesdites images préliminaires successives et notamment en moyennant les valeurs des pixels desdites images préliminaires).

L'intercorrélation susmentionnée peut être réalisée par exemple dans le circuit DSP appartenant à la baie 35, ou être programmée dans l'unité centrale 34 ou dans le micro-ordinateur 27.

Au cours de ce processus d'intercorrélation, on maximise par exemple une fonction d'intercorrélation <Sj(x,y), Sj+1(x,y)> afin de déterminer le déplacement subi par chaque particule 5 donnant lieu à un écho ultrasonore.

Des exemples de tels calculs d'intercorrélation sont donnés dans l'état de la technique, notamment par O'Donnell et al. ("Internal displacement and strain imaging using speckle tracking", IEEE transactions on ultrasonic, ferroelectrics, and frequency control, vol. 41, n° 3, mai 1994, p. 314-325) et par Ophir et al. ("Elastography : a quantitative method for imaging the elasticity of biological tissues", Ultrasonic imag., vol. 13, p.111-134, 1991).

En généralisant la technique d'intercorrélation selon la méthode proposée par Tanter et al (M. Tanter, J. Bercoff, L. Sandrin, M. Fink « Ultrafast compound imaging for 2D motion vector estimation : Application to transient elastography »IEEE Trans. Ultrason., Ferroelec., Freq. Contr. 49 (10), pp 1363-1374, 2002), pour chaque position du réseau 9 de transducteurs, on obtient ainsi un ensemble de vecteurs déplacements $\overrightarrow{u(r,t)}$ engendrés par les ondes de cisaillement en chaque position $\vec{r}$ du champ d'observation dans le plan d'analyse (D, Z) sous l'effet de l'onde de cisaillement (ces vecteurs déplacement sont à deux dimensions et présentent une composante selon la direction D et une composante selon la direction Z).

On peut également effectuer la mesure ou affiner la technique précédente de mesure des déplacements (ou élargir le champ d'observation) dans le plan d'analyse (D, Z) en procédant à une autre série de tirs d'ondes ultrasonores en modifiant le site du réseau 9 de transducteurs par pivotement dudit réseau autour de l'axe R susmentionné.

On répète ensuite la mesure des déplacements dans plusieurs plans d'analyse parallèles (D, Z), en déplaçant le réseau 9 de transducteurs dans plusieurs positions successives le long de la réglette 20. On peut ainsi mesurer les déplacements par exemple dans environ 30 plans parallèles dans le cas de l'imagerie du sein, en une durée totale d'environ 1 à 2 s pour l'ensemble des plans parallèles.

On fait ensuite tourner le plateau 15 d'un angle prédéterminé, ce qui fait également tourner le plan d'analyse (D, Z), et on répète les opérations susmentionnées de mesure des déplacements plusieurs plans d'analyse parallèles à la nouvelle direction D, comme expliqué ci-dessus. On obtient ainsi des champs de déplacements $\overline{u(r,t)}$ à deux dimensions, avec une composante selon la nouvelle direction D et une composante selon la direction Z.

Après avoir effectué les mesures de déplacements avec au moins deux orientations de la direction D (par exemple deux orientations à 90 degrés l'une de l'autre), ou le cas échéant plus de deux orientations (par exemple jusqu'à 36 orientations angulaires), on peut reconstituer un champ de déplacements à trois dimensions $\overline{u(r,t)}$ dans le milieu viscoélastique 2.

Pour cela, on collecte les valeurs de déplacements par exemple selon chaque axe X, Y, Z en fonction du temps, le cas échéant après recalage temporel des différentes mesures (à cet effet, on peut faire émettre un signal avec une modulation périodique par le transducteur 10, et prendre pour origine des temps un même point de la modulation périodique avant chaque séquence de tirs d'ondes ultrasonores) puis on combine ces valeurs par moyennes et interpolations en un seul champ de vecteurs déplacements à trois dimensions $\overrightarrow{u(r,t)}$.

On corrige ensuite ce champ de déplacements $\overline{u(r,t)}$ (ou autre paramètre de mouvement) pour en éliminer les effets dus à la composante de compression de l'onde mécanique élastique générée par le transducteur acoustique 10. A cet effet, on applique au champ $\overrightarrow{u(r,t)}$ l'opérateur rotationnel, pour déterminer un champ vectoriel $\overrightarrow{q(r,t)}=\overrightarrow{rot(u(r,t))}$.

Ainsi, on ne prend en compte que les effets de la composante de cisaillement de l'onde mécanique élastique générée par le transducteur acoustique 10.

Cet ensemble de vecteurs $\overrightarrow{q(r,t)}$ est stocké dans la mémoire M ou dans le micro-ordinateur 4 et peut par exemple être visualisé, notamment au moyen de l'écran 4a du micro-ordinateur, sous la forme d'un film ralenti où la valeur des composantes de q est illustrée par un paramètre optique tel que par un niveau de gris ou par un niveau chromatique.

On visualise ainsi parfaitement les différences de propagation de l'onde de cisaillement entre les zones de caractéristiques différentes du milieu 2, par exemple les tissus sains et les tissus cancéreux dans le cas d'une application médicale.

Ce film de propagation de l'onde de cisaillement est en outre superposable avec une image échographique classique, qui peut être générée également par le dispositif 1 décrit ci-dessus.

Par ailleurs, il est également possible de calculer non pas les déplacements de chaque point du milieu observé 2, mais les déformations du milieu 2, c'est à dire des vecteurs dont les composantes sont les dérivées des composantes des vecteurs déplacements respectivement par rapport aux variables d'espace. Ces vecteurs de déformation sont utilisables comme les vecteurs déplacements pour visualiser clairement la propagation de l'onde de cisaillement sous la forme d'un film, et présentent en outre l'avantage de s'affranchir des déplacements de la sonde 6 par rapport au milieu observé 2. Ces vecteurs déformation sont traités par application de l'opérateur rotationnel pour éliminer les effets dus à la composante de compression de l'o,de mécanique élastique générée par le transducteur acoustique 10, comme expliqué ci-dessus pour les vecteurs déplacements.

A partir des champs de déplacements ou de déformations, le micro-ordinateur 27 peut avantageusement procéder ensuite à une étape de cartographie (d) au cours de laquelle, à partir de l'évolution du paramètre de mouvement (déplacement, vitesse de déplacement ou déformation) traité par application de l'opérateur rotationnel, on calcule au moins un paramètre de propagation des ondes de cisaillement dans le milieu 2.

Le paramètre de propagation de l'onde de cisaillement qui est calculé au cours de l'étape de cartographie est choisi par exemple parmi : la vitesse $C_T$ des ondes de cisaillement, le module de cisaillement $\mu$ ou le module d'Young $E=3\mu$, l'atténuation des ondes de cisaillement, l'élasticité de cisaillement, la viscosité de cisaillement $\zeta$, ou le temps de relaxation mécanique des tissus, ou encore plusieurs de ces paramètres dans le cas d'un milieu anisotrope.

Par exemple, on peut calculer en différents points du champ d'observation $\mu$ le module de cisaillement et $\zeta$ la viscosité de cisaillement.

Pour cela, on peut utiliser l'équation de propagation (1) suivante (dans l'hypothèse où le milieu 2 peut être considéré en première approximation comme un milieu viscoélastique localement homogène et isotrope), à laquelle obéissent les déplacements $\overline{u(r,t)}$ engendrés par les ondes mécaniques élastiques en chaque position $\vec{r}$ du milieu 2 :

$$\rho\,\partial_t^2\vec{u} \;=\; \mu\nabla^2\vec{u} + (\lambda+\mu)\nabla(\nabla\vec{u}) + \varsigma\,\partial_t\nabla^2\vec{u} + (\xi+\varsigma)\partial_t\nabla(\nabla\vec{u}) \qquad , \qquad (1)$$

avec $\rho$ la densité du milieu, $\mu$ le module de cisaillement (que nous cherchons à reconstruire), $\lambda$ le second coefficient de Lamé, $\zeta$ la viscosité de cisaillement prenant en compte la dissipation dans le milieu et $\xi$ la viscosité de l'onde de compression. Le module de cisaillement $\mu$ et le second coefficient de Lamé $\lambda$ sont reliés au module d'Young E et au module de Poisson $\sigma$ par les relations :

$$\mu = \frac{E}{2(1+\sigma)} \quad , \quad \lambda = \frac{\sigma E}{(1+\sigma)(1-2\sigma)} \quad . \qquad (2)$$

Chaque champ vectoriel $(\vec{u})$ peut être décomposé en une partie irrotationnelle $(\vec{u}_L)$, une partie incompressible

$(\vec{u}_T)$ et une partie harmonique $(\vec{u}_H)$, qui est à la fois irrotationnelle et incompressible. La partie harmonique $\vec{u}_H$ prend en compte les déplacements d'ensemble et peut donc être négligée car nous étudions ici le cas d'ondes. En utilisant cette décomposition, il est possible de montrer que l'équation (1) se sépare en deux équations traduisant la propagation des ondes de cisaillement et de compression avec leur vitesses respectives de propagation $c_{T/L}$ (en ignorant l'effet de la viscosité), c'est à dire :

$$\rho\,\partial_t^2\vec{u}_T = \mu\nabla^2\vec{u}_T \qquad c_T = \sqrt{\frac{\mu}{\rho}}$$
$$\rho\,\partial_t^2\vec{u}_L = (\lambda+2\mu)\nabla^2\vec{u}_L \quad,\quad c_L = \sqrt{\frac{\lambda+2\mu}{\rho}} \qquad . \tag{3}$$

En appliquant l'opérateur rotationnel à l'équation (1), on obtient donc une équation d'onde simplifiée qui ne contient plus de contribution de la composante de compression de l'onde mécanique élastique générée par le transducteur 10 :

$$\rho\,\partial_t^2\vec{q} = \mu\nabla^2\vec{q} + \partial_t\varsigma\,\nabla^2\vec{q} \quad,\quad \vec{q} = \nabla\times\vec{u} \quad,\quad \vec{q}\in C^3 \qquad . \tag{4}$$

A partir de l'équation (4) On peut ensuite calculer paramètres $\mu$ et $\zeta$ au prix, maintenant, de dérivées spatiales du troisième ordre. L'équation (4) peut être résolue simplement, par exemple par multiplication par la matrice transposée du système (pseudo-inverse). Il convient de noter que l'équation (5) représente en réalité 6 équations (3 pour la partie réelle et 3 pour la partie imaginaire de $\vec{q}$, en notation complexe) pour seulement deux inconnues (la densité $\rho$ est considérée comme constante et égale à celle de l'eau).

Ainsi, dans le cas du régime permanent, avec les notations :

$$q_i^R = A_i\cos(\omega t + \varphi_i)\big|_{t=0}\,,\quad q_i^I = A_i\cos(\omega t + \varphi_i)\big|_{t=\pi/(2\omega)}\,,\quad i=x,y,z \tag{5}$$

les équations disponibles peuvent s'écrire :

$$-\rho\omega^2\begin{pmatrix} q_x^R \\ q_y^R \\ q_z^R \\ q_x^I \\ q_y^I \\ q_z^I \end{pmatrix} = \begin{pmatrix} \nabla^2 q_x^R & \nabla^2 q_x^I \\ \nabla^2 q_y^R & \nabla^2 q_y^I \\ \nabla^2 q_z^R & \nabla^2 q_z^I \\ \nabla^2 q_x^I & -\nabla^2 q_x^R \\ \nabla^2 q_y^I & -\nabla^2 q_y^R \\ \nabla^2 q_z^I & -\nabla^2 q_z^R \end{pmatrix}\begin{pmatrix} \mu \\ \omega\varsigma \end{pmatrix} \tag{6}.$$

Une multiplication par la matrice transposée donne :

$$-\rho\omega^2\begin{pmatrix} q_i^R\cdot\nabla^2 q_i^R + q_i^I\cdot\nabla^2 q_i^I \\ q_i^R\cdot\nabla^2 q_i^I - q_i^I\cdot\nabla^2 q_i^R \end{pmatrix} = \begin{pmatrix} \nabla^2 q_i^R\cdot\nabla^2 q_i^R + \nabla^2 q_i^I\cdot\nabla^2 q_i^I & 0 \\ 0 & \nabla^2 q_i^I\cdot\nabla^2 q_i^I + \nabla^2 q_i^R\cdot\nabla^2 q_i^R \end{pmatrix}\begin{pmatrix} \mu \\ \omega\varsigma \end{pmatrix} \tag{7}$$

avec *i=x,y,z* et la convention d'Einstein pour les indices identiques. Dans le cas du régime stationnaire, il n'y a pas de corrélation entre $\mu$ et $\zeta$, ce qui rend plus facile la cartographie de ces deux paramètres calculés alors indépendamment l'un de l'autre.

On notera que des calculs analogues peuvent permettre de déterminer les paramètres de propagation recherchés même lorsqu'on ne peut pas faire l'approximation d'un milieu localement homogène et isotrope. Dans ce cas, on utilise une équation e propagation plus complexe :

$$\rho \, \partial_t^2 u_i = \lambda_{iklm} \frac{\partial^2 u_m}{\partial x_k \partial x_l} + \eta_{iklm} \frac{\partial^3 u_m}{\partial_t \, \partial x_k \partial x_l} \quad , \qquad (1')$$

où $u_i$ est le composant n° i du vecteur de déplacement $\overrightarrow{u(r,t)}$, $\rho$ la densité du milieu 2, $\lambda_{iklm}$ le tenseur d'élasticité de rang 4, et $\eta_{iklm}$ le tenseur de viscosité de rang 4 (avec la convention d'Einstein pour les indices identiques).

Cette équation (1') se simplifie dans l'hypothèse d'un milieu transversalement isotrope, c'est à dire d'un milieu présentant des fibres alignées selon une seule direction. Dans ce cas, on peut diviser les paramètres décrivant le matériau en deux groupes :

- un groupe comprenant deux modules de cisaillement ($\mu_\square, \mu_\perp$) décrit la propagation des ondes de cisaillement parallèlement ($\square$) et perpendiculairement ($\perp$) à la direction des fibres,
- l'autre groupe ($\lambda_\square, \lambda_\perp, \lambda_M$) décrit la propagation of des ondes longitudinales (ie les ondes de compression) dans les différentes directions.

La relation entre le tenseur des déformations et le tenseur des contraintes est donnée ici par l'équation :

$$
\begin{pmatrix} \sigma_{xx} \\ \sigma_{yy} \\ \sigma_{zz} \\ \sigma_{yz} \\ \sigma_{xz} \\ \sigma_{xy} \end{pmatrix} = \begin{pmatrix} \lambda_\perp + 2\mu_\perp & \lambda_\perp & \lambda_M & 0 & 0 & 0 \\ \lambda_\perp & \lambda_\perp + 2\mu_\perp & \lambda_M & 0 & 0 & 0 \\ \lambda_M & \lambda_M & \lambda_\square + 2\mu_\square & 0 & 0 & 0 \\ 0 & 0 & 0 & \mu_\square & 0 & 0 \\ 0 & 0 & 0 & 0 & \mu_\square & 0 \\ 0 & 0 & 0 & 0 & 0 & \mu_\perp = \dfrac{C_{11} - C_{12}}{2} \end{pmatrix} \begin{pmatrix} u_{xx} \\ u_{yy} \\ u_{zz} \\ 2u_{yz} \\ 2u_{xz} \\ 2u_{xy} \end{pmatrix} ,
$$

$$(8)$$

où $u_{ik}=1/2(\partial_{x_k}u_i + \partial_{x_i}u_k)$ est le tenseur des déformations et $\sigma_{ik}$ le tenseur des contraintes.

L'équation correspondante de propagation pour le vecteur des déplacements ***u*** s'écrit :

$$\rho \, \partial_t^2 u_i = \frac{\partial \sigma_{ik}}{\partial x_k} + \zeta \partial_t \nabla^2 u_i + \underbrace{(\xi + \zeta) \frac{\partial^3 u_k}{\partial_t \, \partial x_i \partial x_k}}_{\square 1} \quad , $$

$$(9)$$

où $\zeta$ est la viscosité de cisaillement. La viscosité des ondes longitudinales ($\xi$) peut être négligée à relativement basse fréquence (domaine du Hz ou du kHz). De plus, $\nabla u$ a une faible valeur du fait de la valeur du coefficient de Poisson. Ainsi, $\xi$, $\zeta$, et $\nabla \boldsymbol{u}$ ont de faibles valeurs et on peut donc légitimement négliger la troisième partie du côté droit de l'équation 9. En reportant l'équation 8 dans l'équation 9, on obtient :

$$\rho\,\partial_t^2\,\boldsymbol{u} = \mu_\perp \nabla^2 \boldsymbol{u} + \left(\lambda + \mu_\perp\right)\nabla\left(\nabla \boldsymbol{u}\right) + \tau \begin{pmatrix} \dfrac{\partial^2 u_x}{\partial z^2} + \dfrac{\partial^2 u_z}{\partial x \partial z} \\[2mm] \dfrac{\partial^2 u_y}{\partial z^2} + \dfrac{\partial^2 u_z}{\partial y \partial z} \\[2mm] \nabla^2 u_z + \partial_z(\nabla \boldsymbol{u}) \end{pmatrix}$$

$$+ \zeta\,\partial_t\,\nabla^2 \boldsymbol{u}, \quad (10)$$

avec la notation $\tau = \mu_\square - \mu_\perp$ et en supposant que tous les modules élastiques des ondes longitudinales sont égaux, i.e. $\lambda = \lambda_\square = \lambda_\perp = \lambda_M$.

Après application de l'opérateur rotationnel, on obtient :

$$\rho\,\partial_t^2\,\mathbf{q} = \mu_\perp \nabla^2 \mathbf{q} + \tau\,\nabla \times \begin{pmatrix} \dfrac{\partial^2 u_x}{\partial z^2} + \dfrac{\partial^2 u_z}{\partial x \partial z} \\[2mm] \dfrac{\partial^2 u_y}{\partial z^2} + \dfrac{\partial^2 u_z}{\partial y \partial z} \\[2mm] \nabla^2 u_z + \partial_z(\nabla \mathbf{u}) \end{pmatrix} + \zeta\,\partial_t\,\nabla^2 \mathbf{q} \qquad (11)\,.$$

Cette équation 11 donne en fait six équations utilisables pour calculer notamment $\mu_\perp, \tau, \zeta$.

On notera que l'invention serait applicable quelle que soit la méthode de mesure du paramètre de mouvement (déplacement ou autre) du milieu 2. Ce paramètre pourrait ainsi être le cas échéant déterminé par IRM.

Le procédé qui vient d'être décrit pourrait également être couplé à une imagerie acousto-optique du milieu 2, telle que décrite par exemple dans le document WO-A-04/85978.

## Revendications

1. Procédé d'imagerie dans lequel on génère, dans un milieu viscoélastique (2), une onde mécanique ayant une composante de cisaillement et une composante de compression, et on détermine au moins un paramètre de mouvement du milieu viscoélastique (2) en différents points lors de la propagation de cette onde mécanique, ce procédé comportant au moins une étape d'observation (b) au cours de laquelle on observe la propagation de l'onde mécanique en une multitude de points du milieu viscoélastique (2), par une technique choisie parmi l'échographie ultrasonore et la résonance magnétique, et **caractérisé en ce que** l'onde mécanique est entretenue, et **en ce que** ledit procédé comporte une étape de correction au cours de laquelle on traite le paramètre de mouvement pour éliminer les effets dus à la composante de compression de ladite onde mécanique et pour ne prendre en compte que les effets dus à la composante de cisaillement de ladite onde mécanique.

2. Procédé d'imagerie selon la revendication 1, dans lequel au cours de ladite étape de correction, on calcule un rotationnel dudit paramètre de mouvement.

3. Procédé d'imagerie selon la revendication 1, dans lequel l'étape d'observation (b) comprend les sous-étapes suivantes :

   (b1) faire émettre dans le milieu viscoélastique (2), par un réseau (9) de transducteurs commandés indépendamment les uns des autres, une succession de tirs d'ondes ultrasonores de compression à une cadence d'au moins 10 tirs par seconde,

(b2) faire détecter et enregistrer en temps réel des signaux acoustiques reçus du milieu viscoélastique (2), comprenant les échos générés par les ondes ultrasonores de compression en interagissant avec le milieu viscoélastique.

**4.** Procédé d'imagerie selon la revendication 3, comportant en outre au moins une étape de traitement (c) au cours de laquelle :

(c1) on traite les signaux acoustiques successifs reçus du milieu viscoélastique au cours de la sous-étape (b2) pour déterminer des images de propagation successives de l'onde mécanique,

(c2) et on détermine ledit paramètre de mouvement du milieu viscoélastique en différents points à partir desdites images de propagation successives.

**5.** Procédé d'imagerie selon l'une quelconque des revendications précédentes, dans lequel on réalise plusieurs images successives de propagation de l'onde mécanique dans le milieu viscoélastique (2) selon plusieurs points de vue, et on combine ces images successives pour déterminer une valeur vectorielle dudit paramètre de mouvement.

**6.** Procédé d'imagerie selon la revendication 5, dans lequel on détermine une valeur vectorielle à trois dimensions dudit paramètre de mouvement.

**7.** Procédé d'imagerie selon la revendication 5 ou la revendication 6, dans lequel, pour réaliser les images successives de propagation de l'onde mécanique selon plusieurs points de vue, on utilise un réseau (9) de transducteurs ultrasonores qui émet et détecte les ondes ultrasonores de compression selon une direction d'émission et détection (D) dans un plan d'analyse (D, Z), et l'on déplace ledit réseau linéaire (9) à l'extérieur du milieu viscoélastique (2) en faisant tourner ledit plan d'analyse (D, Z).

**8.** Procédé d'imagerie selon la revendication 7, dans lequel on déplace le réseau linéaire (9) en translation sans faire tourner le plan d'analyse (D, Z), puis on fait tourner ledit réseau linéaire (9) en faisant tourner ledit plan d'analyse.

**9.** Procédé d'imagerie selon la revendication 8, dans lequel on fait varier la direction d'émission et réception (D) des transducteurs du réseau sans faire translater ni tourner le plan d'analyse (D, Z), puis on fait translater et tourner ledit réseau linéaire (9) en faisant translater et tourner ledit plan d'analyse.

**10.** Procédé d'imagerie selon l'une quelconque des revendications précédentes, dans lequel ledit paramètre de mouvement est choisi parmi un déplacement, une vitesse de déplacement et une déformation du milieu viscoélastique (2).

**11.** Procédé d'imagerie selon l'une quelconque des revendications précédentes, comprenant en outre une étape (d) de cartographie au cours de laquelle, à partir d'une évolution du paramètre de mouvement au cours du temps, on calcule au moins un paramètre de propagation de la composante de cisaillement de l'onde mécanique en au moins certains points du milieu viscoélastique (2) pour déterminer ainsi une cartographie dudit paramètre de propagation dans le milieu viscoélastique.

**12.** Procédé d'imagerie selon la revendication 11, dans lequel le paramètre de propagation de l'onde de cisaillement qui est calculé au cours de l'étape de cartographie, est choisi parmi la vitesse des ondes de cisaillement, le module de cisaillement, le module d'Young, l'atténuation des ondes de cisaillement, l'élasticité de cisaillement, la viscosité de cisaillement et le temps de relaxation mécanique, ou plusieurs de ces paramètres dans le cas d'un milieu anisotrope.

**13.** Procédé d'imagerie selon l'une quelconque des revendications précédentes, dans lequel le milieu viscoélastique (2) est un corps vivant.

**14.** Dispositif d'imagerie par ultrason ou par résonance magnétique comprenant des moyens pour générer, dans un milieu viscoélastique (2), une onde mécanique ayant une composante de cisaillement et une composante de compression, et des moyens pour déterminer au moins un paramètre de mouvement du milieu viscoélastique en différents points lors de la propagation de cette onde mécanique, ce dispositif comportant des moyens d'observation pour observer la propagation de l'onde mécanique en une multitude de points du milieu viscoélastique (2), par une technique choisie parmi l'échographie ultrasonore et la résonance magnétique, et **caractérisé en ce que** l'onde mécanique est entretenue et **en ce qu'**il comporte des moyens de correction (27) pour traiter le paramètre de mouvement pour éliminer les effets dus à la composante de compression de ladite onde mécanique et pour ne prendre en compte que les effets dus à la composante de cisaillement de ladite onde mécanique.

15. Dispositif d'imagerie selon la revendication 14 ou la revendication 15, dans lequel les moyens d'observation comportent un réseau (9) de transducteurs ultrasonores commandé pour observer la propagation de l'onde mécanique en une multitude de points du milieu viscoélastique (2), par échographie.

16. Dispositif d'imagerie selon la revendication 14, dans lequel les moyens de correction (27) sont adaptés pour calculer un rotationnel dudit paramètre de mouvement.

17. Dispositif d'imagerie selon la revendication 15, comportant des moyens (27, 34) pour :

(b1) faire émettre dans le milieu viscoélastique (2), par ledit réseau (9) de transducteurs, une succession de tirs d'ondes ultrasonores de compression à une cadence d'au moins 10 tirs par seconde,
(b2) faire détecter et enregistrer en temps réel des signaux acoustiques reçus du milieu viscoélastique (2), comprenant les échos générés par les ondes ultrasonores de compression en interagissant avec les particules réfléchissantes dudit milieu viscoélastique.

18. Dispositif d'imagerie selon la revendication 17, dans lequel le dispositif comporte des moyens de traitement (34, 38, 27) adaptés pour :

(c1) traiter les signaux acoustiques successifs reçus du milieu viscoélastique pour déterminer des images de propagation successives de l'onde mécanique,
(c2) et déterminer ledit paramètre de mouvement du milieu viscoélastique en différents points du champ d'observation à partir desdites images de propagation successives.

19. Dispositif d'imagerie selon la revendication 18, dans lequel les moyens de traitement (34, 38, 27) sont adaptés pour réaliser plusieurs images successives de propagation de l'onde mécanique dans le milieu viscoélastique (2) selon plusieurs points de vue, pour combiner ces images successives en déterminant une valeur vectorielle dudit paramètre de mouvement.

20. Dispositif d'imagerie selon la revendication 19, dans lequel les moyens de traitement sont adaptés pour déterminer une valeur vectorielle à trois dimensions dudit paramètre de mouvement.

21. Dispositif d'imagerie selon la revendication 19 ou la revendication 20, dans lequel le réseau de transducteurs (9) émet et détecte les ondes ultrasonores de compression selon une direction d'émission et détection (D) dans un plan d'analyse (D, Z) et, pour réaliser les images successives de propagation de l'onde mécanique selon plusieurs points de vue, le dispositif comporte des moyens de déplacement (30, 31, 32, 22, 33) dudit réseau linéaire (9) à l'extérieur du milieu viscoélastique (2), adaptés pour faire tourner ledit plan d'analyse (D, Z).

22. Dispositif d'imagerie selon la revendication 21, comportant des moyens de commande (27) adaptés pour commander les moyens de déplacement (30, 31, 32, 22, 33) et lesdits moyens de commande sont adaptés pour déplacer le réseau (9) en translation sans faire tourner le plan d'analyse (D, Z), puis pour faire tourner ledit réseau (9) en faisant tourner ledit plan d'analyse.

23. Dispositif d'imagerie selon la revendication 21 ou la revendication 22, comportant des moyens de commande (27) adaptés pour commander les moyens de déplacement (30, 31, 32, 22, 33) et lesdits moyens de commande (27) sont adaptés pour faire varier la direction d'émission et réception (D) des transducteurs du réseau (9) sans faire translater ni tourner le plan d'analyse (D, Z), puis pour faire translater et tourner ledit réseau linéaire (9) en faisant translater et tourner ledit plan d'analyse (D, Z).

24. Dispositif d'imagerie selon l'une quelconque des revendications 21 à 23, comportant des moyens de cartographie (27) adaptés pour calculer au moins un paramètre de propagation de la composante de cisaillement de l'onde mécanique en au moins certains points du milieu viscoélastique (2) pour déterminer ainsi une cartographie dudit paramètre de propagation dans le milieu viscoélastique.

**Patentansprüche**

1. Bildgebungsverfahren, bei dem in einem viskoelastischen Medium (2) eine mechanische Welle mit einer Scher-Komponente und einer Druck-Komponente erzeugt wird und wenigstens ein Bewegungsparameter von dem viskoelastischen Medium (2) in unterschiedlichen Punkten bei der Ausbreitung dieser mechanischen Welle bestimmt wird, wobei das Verfahren wenigstens einen Beobachtungsschritt (b) umfasst, bei dem die Ausbreitung der mechanischen Welle in einer Mehrzahl von Punkten des viskoelastischen Mediums (2) durch eine Technik beobachtet wird, die ausgewählt ist aus der Ultraschall-Echographie und der Magnetresonanz, und

   **dadurch gekennzeichnet, dass** die mechanische Welle ungedämpft ist, und dass das Verfahren einen Korrekturschritt umfasst, bei dem der Bewegungsparameter bearbeitet wird, um die Effekte zu eliminieren, die auf die Druck-Komponente der mechanischen Welle zurückgehen, und um nur die Effekte zu berücksichtigen, die auf die Scher-Komponente der mechanischen Welle zurückgehen.

2. Bildgebungsverfahren nach Anspruch 1, wobei bei dem Korrekturschritt eine Rotation des Bewegungsparameters berechnet wird.

3. Bildgebungsverfahren nach Anspruch 1, wobei der Beobachtungsschritt (b) die folgenden Unterschritte umfasst:

   (b1) Emittieren einer Folge von Ultraschall-Druckwellen-Schüssen in das viskoelastische Medium (2) durch ein Netz (9) von unabhängig voneinander gesteuerten/geregelten Wandlern, mit einem Takt von wenigstens 10 Schüssen pro Sekunde,
   (b2) Erfassen und Aufzeichnen von akustischen Signalen in Echtzeit, die aus dem viskoelastischen Medium (2) erhalten werden, umfassend die Echos, welche durch die Ultraschall-Druckwellen durch Wechselwirkung mit dem viskoelastischen Medium erzeugt werden.

4. Bildgebungsverfahren nach Anspruch 3, weiter umfassend wenigstens einen Bearbeitungsschritt (c), bei dem:

   (c1) die aufeinander folgenden, beim Unterschritt (b2) aus dem viskoelastischen Medium erhaltenen akustischen Signale bearbeitet werden, um aufeinander folgende Ausbreitungsbilder der mechanischen Welle zu bestimmen, und
   (c2) der Bewegungsparameter des viskoelastischen Mediums in verschiedenen Punkten ausgehend von den aufeinander folgenden Ausbreitungsbildern bestimmt wird.

5. Bildgebungsverfahren nach einem der vorhergehenden Ansprüche, wobei mehrere, aufeinander folgende Ausbreitungsbilder der mechanischen Welle in dem viskoelastischen Medium (2) von mehreren Beobachtungspunkten aus erzeugt werden, und die aufeinander folgenden Bilder kombiniert werden, um einen Vektor-Wert von dem Bewegungsparameter zu bestimmen.

6. Bildgebungsverfahren nach Anspruch 5, wobei ein Vektor-Wert in drei Dimensionen von dem Bewegungsparameter bestimmt wird.

7. Bildgebungsverfahren nach Anspruch 5 oder Anspruch 6, wobei, um die aufeinander folgenden Ausbreitungsbilder der mechanischen Welle von mehreren Beobachtungspunkten aus zu erhalten, ein Netz (9) von Ultraschall-Wandlern verwendet wird, welches die Ultraschall-Druckwellen emittiert und erfasst, in einer Emissions- und Detektions-Richtung (D) in einer Analyse-Ebene (D, Z), und das lineare Netz (9) außerhalb von dem viskoelastischen Medium (2) verlagert wird, wobei die Analyse-Ebene (D, Z) gedreht wird.

8. Bildgebungsverfahren nach Anspruch 7, wobei das lineare Netz (9) translatorisch verlagert wird, ohne die Analyse-Ebene (D, Z) zu drehen, und dann das lineare Netz (9) gedreht wird, wobei die Analyse-Ebene gedreht wird.

9. Bildgebungsverfahren nach Anspruch 8, wobei die Emissions- und Empfangs-Richtung (D) der Wandler von dem Netz variiert wird, ohne die Analyse-Ebene (D, Z) zu verschieben oder zu drehen, und dann das lineare Netz (9) verschoben und gedreht wird, wobei die Analyse-Ebene verschoben und gedreht wird.

10. Bildgebungsverfahren nach einem der vorhergehenden Ansprüche, wobei der Bewegungsparameter ausgewählt ist aus einer Verlagerung, einer Verlagerungsgeschwindigkeit und einer Verformung des viskoelastischen Mediums (2).

**11.** Bildgebungsverfahren nach einem der vorhergehenden Ansprüche, weiterhin umfassend einen Kartographie-Schritt (d), bei dem, ausgehend von einer Entwicklung des Bewegungsparameters mit der Zeit, wenigstens ein Ausbreitungsparameter der Scher-Komponente der mechanischen Welle wenigstens in bestimmten Punkten des viskoelastischen Mediums (2) berechnet wird, um so eine Kartographie von dem Ausbreitungsparameter in dem viskoelastischen Medium zu bestimmen.

**12.** Bildgebungsverfahren nach Anspruch 11, wobei der Ausbreitungsparameter der Scher-Welle, der bei dem Kartographie-Schritt berechnet wird, ausgewählt ist aus der Geschwindigkeit der Scher-Wellen, dem Scher-Modul, dem Young-Modul, der Dämpfung der Scher-Wellen, der Scher-Elastizität, der Scher-Viskosität und der Zeit der mechanischen Relaxation, oder mehreren dieser Parameter im Fall eines anisotropen Mediums.

**13.** Bildgebungsverfahren nach einem der vorhergehenden Ansprüche, wobei das viskoelastische Medium (2) ein lebender Körper ist.

**14.** Bildgebungseinrichtung mit Ultraschall oder Magnetresonanz, umfassend Mittel, um in einem viskoelastischen Medium (2) eine mechanische Welle zu erzeugen, die eine Scher-Komponente und eine Druck-Komponente aufweist, und Mittel, um wenigstens einen Bewegungsparameter von dem viskoelastischen Medium in verschiedenen Punkten bei der Ausbreitung dieser mechanischen Welle zu bestimmen, wobei die Einrichtung Beobachtungsmittel umfasst, um die Ausbreitung der mechanischen Welle in einer Mehrzahl von Punkten des viskoelastischen Mediums (2) durch eine Technik zu beobachten, die ausgewählt ist aus der Ultraschall-Echographie und der Magnetresonanz, und **dadurch gekennzeichnet, dass** die mechanische Welle ungedämpft ist, und dass Korrekturmittel (27) vorgesehen sind, um den Bewegungsparameter zu bearbeiten, um die Effekte zu eliminieren, welche auf die Druck-Komponente der mechanischen Welle zurückgehen, und um nur die Effekte zu berücksichtigen, welche auf die Scher-Komponente der mechanischen Welle zurückgehen.

**15.** Bildgebungseinrichtung nach Anspruch 14 oder Anspruch 15, wobei die Beobachtungsmittel ein Netz (9) von Ultraschall-Wandlern umfassen, das gesteuert/geregelt wird, um die Ausbreitung der mechanischen Welle in einer Mehrzahl von Punkten des viskoelastischen Mediums (2) durch Echographie zu beobachten.

**16.** Bildgebungseinrichtung nach Anspruch 14, wobei die Korrekturmittel (27) dazu angepasst sind, eine Rotation des Bewegungsparameters zu berechnen.

**17.** Bildgebungseinrichtung nach Anspruch 15, umfassend Mittel (27, 34), um:

(b1) in das viskoelastische Medium (2) durch das Netz (9) von Wandlern eine Folge von Ultraschall-Druckwellen-Schüssen mit einem Takt von wenigstens 10 Schüssen pro Sekunde zu emittieren,
(b2) in Echtzeit akustische Signale zu erfassen und aufzuzeichnen, die aus dem viskoelastischen Medium (2) erhalten werden, umfassend die Echos, welche durch die Ultraschall-Druckwellen durch Wechselwirkung mit den reflektierenden Teilchen des viskoelastischen Mediums erzeugt werden.

**18.** Bildgebungseinrichtung nach Anspruch 17, wobei die Einrichtung Bearbeitungsmittel (34, 38, 27) umfasst, die dazu angepasst sind:

(c1) die aufeinander folgenden, akustischen Signale zu bearbeiten, die aus dem viskoelastischen Medium erhalten werden, um aufeinander folgende Ausbreitungsbilder der mechanischen Welle zu bestimmen, und
(c2) den Bewegungsparameter von dem viskoelastischen Medium in verschiedenen Punkten des Beobachtungsfelds ausgehend von den aufeinander folgenden Ausbreitungsbildern zu bestimmen.

**19.** Bildgebungseinrichtung nach Anspruch 18, wobei die Bearbeitungsmittel (34, 38, 27) dazu angepasst sind, mehrere, aufeinander folgende Ausbreitungsbilder der mechanischen Welle in dem viskoelastischen Medium (2) von mehreren Beobachtungspunkten aus zu erzeugen, um die aufeinander folgenden Bilder zu kombinieren wobei ein Vektor-Wert von dem Bewegungsparameter bestimmt wird.

**20.** Bildgebungseinrichtung nach Anspruch 19, wobei die Bearbeitungsmittel dazu angepasst sind, einen Vektor-Wert in drei Dimensionen von dem Bewegungsparameter zu bestimmen.

**21.** Bildgebungseinrichtung nach Anspruch 19 oder Anspruch 20, wobei das Netz von Wandlern (9) die Ultraschall-Druckwellen gemäß einer Emissions- und Detektions-Richtung (D) in einer Analyse-Ebene (D, Z) emittiert und erfasst und wobei, um die aufeinander folgenden Ausbreitungsbilder der mechanischen Welle von mehreren Beobachtungspunkten aus zu erzeugen, die Einrichtung Verlagerungsmittel (30, 31, 32, 22, 33) für das lineare Netz (9) außerhalb von dem viskoelastischen Medium (2) umfasst, die dazu angepasst sind, die Analyse-Ebene (D, Z) zu drehen.

22. Bildgebungseinrichtung nach Anspruch 21, umfassend Steuer-/Regelmittel (27), die dazu angepasst sind, die Verlagerungsmittel (30, 31, 32, 22, 33) zu steuern/regeln, und wobei die Steuer-/Regelmittel dazu angepasst sind, das Netz (9) translatorisch zu verlagern, ohne die Analyse-Ebene (D, Z) zu drehen, und dann das Netz (9) zu drehen, wobei die Analyse-Ebene gedreht wird.

23. Bildgebungseinrichtung nach Anspruch 21 oder Anspruch 22, umfassend Steuer-/Regelmittel (27), die dazu angepasst sind, die Verlagerungsmittel (30, 31, 32, 22, 33) zu steuern/regeln, und wobei die Steuer-/Regelmittel (27) dazu angepasst sind, die Emissions- und Empfangs-Richtung (D) der Wandler von dem Netz (9) zu variieren, ohne die Analyse-Ebene (D, Z) zu verschieben oder zu drehen, und dann das lineare Netz (9) zu verschieben und zu drehen, wobei die Analyse-Ebene (D, Z) verschoben und gedreht wird.

24. Bildgebungseinrichtung nach einem der Ansprüche 21 bis 23, umfassend Kartographie-Mittel (27), die dazu angepasst sind, wenigstens einen Ausbreitungsparameter der Scher-Komponente der mechanischen Welle in wenigstens bestimmten Punkten des viskoelastischen Mediums (2) zu berechnen, um so eine Kartographie des Ausbreitungsparameters in dem viskoelastischen Medium zu bestimmen.

## Claims

1. An imaging method in which a mechanical wave having a shear component and a compression component is generated in a viscoelastic medium (2) and at least one parameter describing the movement of the viscoelastic medium (2) at various points in the propagation of this mechanical wave is determined, the method including at least one observation step (b) during which the propagation of the mechanical wave at a multitude of points in the viscoelastic medium (2) is observed by a technique chosen from ultrasound echography and magnetic resonance, and

   **characterized in that** the mechanical wave is sustained, and **in that** said method includes a correction step during which the movement parameter is processed so as to remove the effects due to the compression component of said mechanical wave and for taking into account only the effects due to the shear component of said mechanical wave.

2. The imaging method as claimed in claim 1, in which a curl of said movement parameter is calculated during said correction step.

3. The imaging method as claimed in claim 1, in which the observation step (b) comprises the following substeps:

   (b1) a succession of shots of ultrasonic compression waves are transmitted into the viscoelastic medium (2) at a rate of at least ten shots per second by an array (9) of transducers controlled independently of one another; and
   (b2) acoustic signals received from the viscoelastic medium (2), including the echoes generated by the ultrasonic compression waves interacting with the viscoelastic medium, are detected and recorded in real time.

4. The imaging method as claimed in claim 3, which furthermore includes at least one processing step (c) during which:

   (c1) the successive acoustic signals received from the viscoelastic medium during substep (b2) are processed in order to determine successive propagation images of the mechanical wave; and
   (c2) said movement parameter of the viscoelastic medium is determined at various points from said successive propagation images.

5. The imaging method as claimed in any one of the preceding claims, in which several successive propagation images of the mechanical wave in the viscoelastic medium (2) are produced along several points of view and these successive images are combined in order to determine a vector value of said movement parameter.

6. The imaging method as claimed in claim 5, in which a three-dimensional vector value of said movement parameter is determined.

7.  The imaging method as claimed in claim 5 or claim 6, in which, in order to produce successive propagation images of the mechanical wave along several points of view, an array (9) of ultrasonic transducers is used that transmits and detects the ultrasonic compression waves along a transmission/detection direction (D) in an analysis plane (D-Z) and said linear array (9) is displaced outside the viscoelastic medium (2), causing said analysis plane (D-Z) to be rotated.

8.  The imaging method as claimed in claim 7, in which the linear array (9) is displaced by moving translationally, without the analysis plane (D-Z) being rotated, and then said linear array (9) is rotated, causing said analysis plane to be rotated.

9.  The imaging method as claimed in claim 8, in which the transmission/reception direction (D) of the transducers of the array is varied without either translating or rotating the analysis plane (D-Z), and then said linear array (9) is translated and rotated, causing said analysis plane to be translated and rotated.

10. The imaging method as claimed in any one of the preceding claims, in which said movement parameter is chosen from a displacement, a displacement velocity and a stress of the viscoelastic medium (2).

11. The imaging method as claimed in any one of the preceding claims, which furthermore includes a mapping step (d) during which at least one parameter describing the propagation of the shear component of the mechanical wave at at least certain points in the viscoelastic medium (2) is calculated, from a variation of the movement parameter over time, in order in this way to determine a map of said propagation parameter in the viscoelastic medium.

12. The imaging method as claimed in claim 11, in which the propagation parameter of the shear wave, which is calculated during a mapping step, is chosen from the velocity of the shear waves, the shear modulus, the Young's modulus, the attenuation of the shear waves, the shear elasticity, the shear viscosity and the mechanical relaxation time, or several of these parameters in the case of an anisotropic medium.

13. The imaging method as claimed in any one of the preceding claims, in which the viscoelastic medium (2) is a living body.

14. An imaging device comprising means for generating, in a viscoelastic medium (2), a mechanical wave having a shear component and a compression component and means for determining at least one parameter describing the movement of the viscoelastic medium at different points during the propagation of this mechanical wave, the device including observation means for observing the propagation of the mechanical wave at a multitude of points in the viscoelastic medium (2) by a technique chosen from ultrasound echography and magnetic resonance, and **characterized in that** the mechanical wave is sustained, and **in that** it includes correction means (27) for processing the movement parameter so as to remove the effects due to the compression component of said mechanical wave and for taking into account only the effects due to the shear component of said mechanical wave.

15. The imaging device as claimed in claim 14, in which the observation means comprise an array (9) of ultrasonic transducers controlled for observing the propagation of the mechanical wave at a multitude of points in the viscoelastic medium (2), by echography.

16. The imaging device as claimed in claim 14, in which the correction means (27) are designed to calculate a curl of said movement parameter.

17. The imaging device as claimed in claim 15, which includes means (27, 34) for:

    (b1) transmitting a succession of shots of ultrasonic compression waves into the viscoelastic medium (2) by said array (9) of transducers at a rate of at least ten shots per second; and
    (b2) detecting and recording acoustic signals received from the viscoelastic medium (2), including the echoes generated by the ultrasonic compression waves interacting with the reflecting particles of said viscoelastic medium, in real time.

18. The imaging device as claimed in claim 17, in which the device includes processing means (34, 38, 27) designed to:

    (c1) process the successive acoustic signals received from the viscoelastic medium in order to determine successive propagation images of the mechanical wave; and

(c2) determine said movement parameter of the viscoelastic medium at various points in the observation field from said successive propagation images.

19. The imaging device as claimed in claim 18, in which the processing means (34, 38, 27) are designed to produce several successive propagation images of the mechanical wave in the viscoelastic medium (2) along several points of view and to combine these successive images for determining a vector value of said movement parameter.

20. The imaging device as claimed in claim 19, in which the processing means are designed to determine a three-dimensional vector value of said movement parameter.

21. The imaging device as claimed in claim 19 or claim 20, in which the array of transducers (9) transmits and detects the ultrasonic compression waves along a transmission/detection direction (D) in an analysis plane (D-Z) and, to produce the successive propagation images of the mechanical wave along several points of view, the device includes displacement means (30, 31, 32, 22, 33) for displacing said linear array (9) outside the viscoelastic medium (2), said means being designed to rotate said analysis plane (D-Z).

22. The imaging device as claimed in claim 21, which includes control means (27) designed to control the displacement means (30, 31, 32, 33) and said control means are designed to displace the array (9) translationally without the analysis plane (D-Z) being rotated, and then to rotate said array (9), causing said analysis plane to be rotated.

23. The imaging device as claimed in claim 21 or claim 22, which includes control means (27) designed to control the displacement means (30, 31, 32, 22, 33), and said control means (27) are designed to vary the transmission/reception direction (D) of the transducers of the array (9) without either translating or rotating the analysis plane (D-Z) and then to translate and rotate said linear array (9), causing said analysis plane (D-Z) to be translated and rotated.

24. The imaging device as claimed in any one of the preceding claims 21 to 23, which includes mapping means (27) designed to calculate at least one parameter describing the propagation of the shear component of the mechanical wave at at least certain points in the viscoelastic medium (2) in order in this way to determine a map of said propagation parameter in the viscoelastic medium.

**FIG. 1**

**FIG. 2**

FIG. 3

## RÉFÉRENCES CITÉES DANS LA DESCRIPTION

**Documents brevets cités dans la description**

- WO 0421038 A
- US 5606971 A
- WO 0485978 A


**Littérature non-brevet citée dans la description**

- **O'DONNELL et al.** Internal displacement and strain imaging using speckle tracking. *IEEE transactions on ultrasonic, ferroelectrics, and frequency control,* Mai 1994, vol. 41 (3), 314-325
- **OPHIR et al.** Elastography : a quantitative method for imaging the elasticity of biological tissues. *Ultrasonic imag.,* vol. 13, 111-134
- **M. TANTER ; J. BERCOFF ; L. SANDRIN ; M. FINK.** Ultrafast compound imaging for 2D motion vector estimation : Application to transient elastography. *IEEE Trans. Ultrason., Ferroelec., Freq. Contr.,* 2002, vol. 49 (10), 1363-1374